# EUROPEAN PATENT SPECIFICATION

(11) **EP 1 131 411 B1**
(45) Date of publication and mention of the grant of the patent: **23.06.2004**
(21) Application number: 99956179.8
(22) Date of filing: 22.11.1999
(51) Int. Cl.: C12N 5/10, C07K 16/46, C12N 15/62, A01K 67/027, C12N 15/00

(54) **SUPPRESSION OF XENOTRANSPLANT REJECTION**
UNTERDRÜCKUNG DER ABSTOSSUNG BEI XENOTRANSPLANTATION
SUPPRESSION DU REJET DE XENOTRANSPLANTS

(30) Priority: 20.11.1998 GB 9825555
(43) Date of publication of application: 12.09.2001
(73) Proprietor: IMPERIAL COLLEGE INNOVATIONS LIMITED, London SW7 2AZ (GB)
(72) Inventor: RAMRAKHA, Punit, Satyavrat, London W9 1HR (GB); GEORGE, Andrew, John, Timothy, Richmond, Surrey TW10 6BW (GB); HASKARD, Dorian, of Medicine, Du Cane Road,London W12 0NN (GB); LECHLER, Robert, Ian, London W4 1EQ (GB); DORLING, Anthony Imperial College School of Med., London W12 0NN (GB)
(74) Representative: Howard, Paul Nicholas
(86) International application number: PCT/GB1999/003888
(87) International publication number: WO 2000/031126

(56) References cited:
- WO-A-96/15780
- WO-A-97/11971
- DORLING ANTHONY ET AL: "In vitro accommodation of immortalized porcine endothelial cells: Resistance to complement mediated lysis and down-regulation of VCAM expression induced by low concentrations of polyclonal human IgG antipig antibodies." TRANSPLANTATION (BALTIMORE) 1996, vol. 62, no. 8, 1996, pages 1127-1136, XP000909707 ISSN: 0041-1337
- GERRITSEN M E ET AL: "FLAVENOIDS INHIBIT CYTOKINE-INDUCED ENDOTHELIAL CELL ADHESION PROTEIN GENE EXPRESSION" AMERICAN JOURNAL OF PATHOLOGY,US,PHILADELPHIA, PA, vol. 147, no. 2, 1 August 1995 (1995-08-01), pages 278-292, XP000575649 ISSN: 0002-9440
- ELICES M J ET AL: "VCAM-1 ON ACTIVATED ENDOTHELIUM INTERACTS WITH THE LEUKOCYTE INTEGRIN VLA-4 AT A SITE DISTINCT FROM THE VLA-4/FIBRONECTIN BINDING SITE" CELL,US,CELL PRESS, CAMBRIDGE, NA, vol. 60, 23 February 1990 (1990-02-23), pages 577-584, XP000606922 ISSN: 0092-8674
- ROBINSON L A ET AL: "The role of adhesion molecules in human leukocyte attachment to porcine vascular endothelium: implications for xenotransplantation." JOURNAL OF IMMUNOLOGY, (1998 DEC 15) 161 (12) 6931-8. , XP000887398
- HAMMER C ET AL: "Organs from animals for man." INTERNATIONAL ARCHIVES OF ALLERGY AND IMMUNOLOGY MAY, 1998, vol. 116, no. 1, May 1998 (1998-05), pages 5-21, XP000909785 ISSN: 1018-2438
- ISOBE M ET AL: "SPECIFIC ACCEPTANCE OF CARDIAC ALLOGRAFT AFTER TREATMENT WITH ANTIBODIES TO ICAM-1 AND LFA1" SCIENCE,US,AMERICAN ASSOCIATION FOR THE ADVANCEMENT OF SCIENCE,, vol. 255, 28 February 1992 (1992-02-28), pages 1125-1127, XP002056591 ISSN: 0036-8075 cited in the application

## Description

The present invention relates to the suppression of graft rejection, particularly to the suppression of xenograft rejection.

The success of allogeneic organ transplantation has become well established in the last few decades. However, the limited supply of donor organs means that many patients have little or no chance of receiving a transplanted organ and thus die before a suitable organ is found. One potential solution to this problem is "xenografting", or the use of organs from a non-human ("xenogeneic") animal donor.

Porcine donor organs are particularly suitable candidates for transplantation because pigs are anatomically and physiologically similar to humans, are in abundant supply and are relatively free of pathogens that are capable of causing infections in humans. Furthermore, transgenic technology affords the possibility of genetically modifying the donor tissue to abrogate the rejection response.

One problem associated with xenografting is that xenogeneic organs are rejected rapidly upon re-vascularisation by a humoral process called hyperacute rejection (HAR). This is caused by the presence of naturally-occurring antibodies in the graft recipient, which recognise and react with antigens on the endothelial cells (ECs) of the xenograft. This recognition triggers the complement cascade which in turn leads to rejection.

In the last few years, several novel therapeutic approaches to suppress HAR have been proposed and tested successfully (Bach, 1998). These work either by suppression of complement activation or preventing binding of xenoreactive natural antibodies. HAR is no longer considered an insurmountable problem for pig-to-human transplantation. However, it is becoming clear that preventing HAR alone is unlikely to be sufficient to prevent rejection of xenogeneic organs.

Even if HAR is overcome, vigorous rejection of the graft typically occurs within 2-3 days, much faster than for most forms of allogeneic transplantation, a process termed delayed xenograft rejection (DXR). The histology of this type of rejection is different from HAR with less haemorrhage although with significant intravascular thrombosis. In addition, there is deposition of xenoreactive antibodies on the endothelium along with fibrin, platelet aggregates and infiltration of the perivascular tissue with inflammatory cells (neutrophils, macrophages and NK cells) (Blakely *et al.,* 1994).

Beyond DXR there is the problem of T-lymphocyte mediated rejection. It has been demonstrated (Dorling *et al.,* 1994) that the T-cell response to porcine xenografts is at least equivalent to the response against allografts, but is likely to be more aggressive and may be difficult to control with standard doses of systemic immunosuppressive drugs.

Endothelial cells (ECs) are thought to orchestrate the recruitment of inflammatory cells in DXR and subsequent cellular rejection in a number of ways: (i) by producing mediators (such as interleukin-8 (IL-8) and platelet activating factor (PAF)) that activate leukocyte function, including adhesion; (ii) by acting as antigen-presenting cells, stimulating the specific immune response against the foreign tissue, and (iii) by regulating the spatial and temporal expression of cell-adhesion molecules to facilitate transmigration of leukocytes into the transplanted organ (Bevilacqua, 1993). Cytokines, released from the recruited leukocytes, dramatically increase the expression of adhesion molecules on the EC surface and enhance the recruitment process.

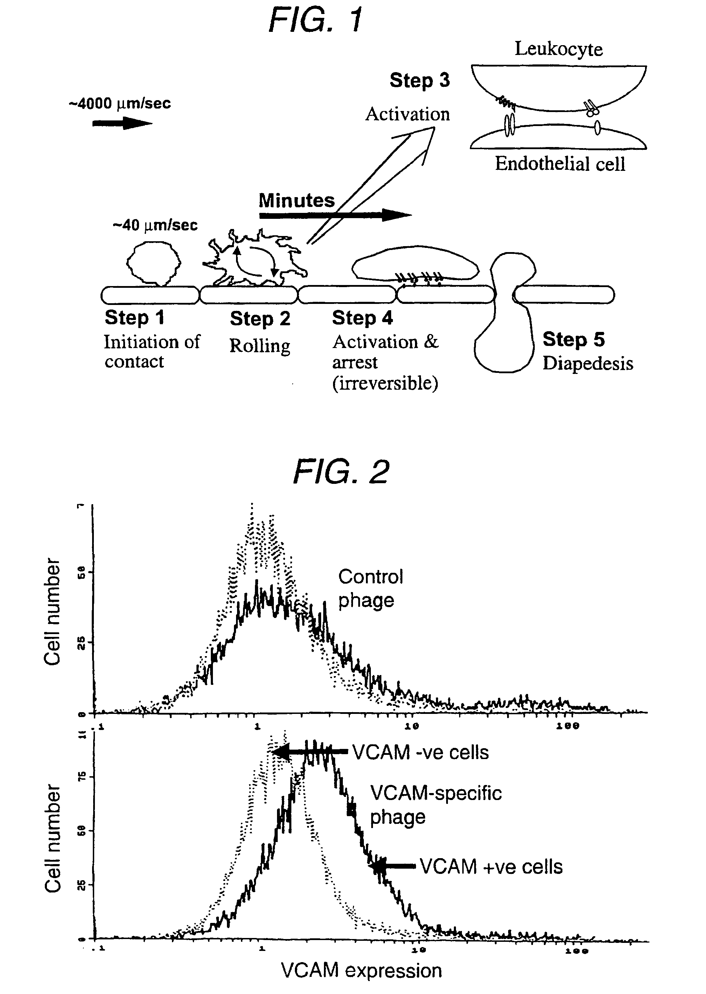

The first structure that circulating leukocytes come in contact with when blood reperfuses a vascularised graft is the endothelium. The leukocytes must adhere to and cross this endothelial barrier to infiltrate the graft. Recent advances in the understanding of mechanisms of leukocyte-EC interactions have revealed a series of adhesion and activation events (the adhesion cascade) that take place during the emigration of leukocytes into tissues (Springer, 1994). (See Figure 1).

Initial rolling on vascular endothelium is mediated by transient interactions between selectins (e.g. L-selectin on leukocytes, E-selectin on activated EC and P-selectin on both activated EC and activated platelets) and carbohydrate-bearing counter-structures on the opposite cell (EC, leukocyte or platelet) (Tedder *et al.,* 1995). However due to the high on/off rate of these selectin-carbohydrate interactions, this class of receptors cannot support firm adhesion of leukocytes. While rolling, leukocytes become exposed to activating signals which result in an increase in avidity of leukocyte surface integrins. Chemokines are thought to be the likely candidates for this triggering event: IL-8 has been shown to exist anchored to the EC surface via surface proteoglycans (Tanaka *et* *al.,* 1993) resulting in high local concentrations within the milieu of the rolling leukocyte. This integrin-mediated secondary adhesion results in stable arrest of the leukocyte and is followed by transmigration into the tissues (Springer, 1994).

Members of the immunoglobulin supergene family (IgSF) expressed on the endothelium are counter-receptors for leukocyte integrins. These include vascular cell adhesion molecule (VCAM-1), intercellular adhesion molecules (ICAM-1, ICAM-2), and mucosal vascular addressin (MAdCAM-1). The counter ligands for VCAM-1 are heterodimeric α4 integrins with a non covalent linkage to either a β1 or β7 chain.

Integrin α4β1 (very late antigen-4 [VLA-4]) is constitutively expressed on most mononuclear leukocytes including eosinophils, lymphocytes, monocytes, basophils but is absent on neutrophils. In contrast, integrin α4β1 is found primarily on a subset of T cells with a tropism for the intestinal tract and its primary ligand is the mucosal vascular addressin (MAdCAM-1), though it also binds VCAM-1 (18). Leukocyte receptors for ICAM are the β2 integrins 'lymphocyte function associated antigen 1' (LFA-1) and αMβ2 integrin (Mac-1). Neutrophils and all haematopoietic cells (except erythrocytes) express LFA-1, whereas Mac-1 expression is more restricted to monocytes, macrophages and granulocytes.

Quiescent vascular endothelium expresses an array of molecules that should not be sufficient to promote significant binding of leukocytes and subsequent transmigration. However it is well established that peri-transplant organ ischaemia results in endothelial activation with increased expression of adhesion molecules.

The interaction of VCAM-1 on ECs with its ligand, the α4β1 integrin VLA-4, on the leukocyte has recently been shown to be the predominant mechanism triggering arrest of rolling monocytes and lymphocytes, and subsequent spreading (Jones *et al.,* 1994; Alon *et al.,* 1995). Transmigration through endothelial tight junctions involves platelet-endothelial cell adhesion molecule (PECAM, CD31), integrin associated protein (IAP, CD47) and integrin α4β1 (Muller, 1995; Brown, 1996).

It appears that interfering with the recognition processes mediated by these adhesion molecules (in particular ICAM-1, LFA-1, VCAM-1 and CD2) may significantly prolong allogeneic graft survival. Furthermore in some models, not only did adhesion molecule blockade with monoclonal antibodies induce indefinite graft survival, but also donor-specific tolerance (Isobe *et al.,* 1992).

Porcine ECs have the capacity to mediate both the initial adhesion as well as the migration and activation of infiltrating human leukocytes. Functional interactions between human LFA-1 and pig ICAM, and human VLA-4 and pig VCAM have been documented. Furthermore interaction of human monocytes with porcine endothelium also results in activation of the ECs (Millan *et al.,* 1997). This is likely to promote trafficking of human lymphocytes and monocytes into a porcine xenograft and trigger rejection.

It has been demonstrated that antibodies against porcine adhesion molecules can inhibit the infiltration process (Dorling *et al.,* 1996; Mueller *et al.,* 1995). To inhibit the interaction of VCAM-1 and VLA-4, monoclonal antibodies against either of these molecules have been administered. A VCAM-Ig fusion protein, cyclic peptide antagonists that mimic the α4-integrin binding loop in domain 1 of VCAM-1, and certain naturally-occurring fungal cyclopeptolides have also been used (reviewed in Foster, 1996).

It might be possible to target porcine VCAM-1 specifically with monoclonal antibodies, but it is thought that repeated administration of these antibodies would result in sensitization of the recipient and a decline in efficiency of blockade. Systemic administration of agents such as the VCAM-Ig fusion protein, cyclic peptide antagonists naturally-occurring fungal cyclopeptolides mentioned above would result in blockade not only of the pVCAM-VLA-4 interaction within the graft but also in other tissues of the recipient and may impair the ability of the recipient to fight infections.

There is thus a great need for a method of combating the cellular phase of the rejection process resulting from xenotransplantation, without compromising the immune system of the recipient of the grafted tissue.

### SUMMARY OF THE INVENTION

According to the present invention there is provided a biological tissue comprising endothelial cells which may be induced to generate a compound which down-regulates the expression of a cell adhesion molecule by the cells.

The biological tissue may be any tissue suitable for transplantation to a mammal, and includes collections of cells, and individual tissues and organs. Accordingly, this definition includes, fibroblasts, neural tissue, foetal tissue and heart, liver, lung , pancreas, islets, skin, small bowel, cornea, cartilage, bone, muscle, or kidney tissues or organs.

The tissue may be derived from any non-human animal which is sufficiently closely related to the human to allow conservation of function to have been retained. Such animals include sheep, pigs, ratites (ostrich, emu), capybara and primates. The animal of choice is the pig, because their organs have similar physiology and size to human organs. Furthermore, pigs can be bred in large numbers and they are relatively free of pathogens capable of causing infections in humans.

As used herein, the term "expression" may refer to the expression of a peptide or polypeptide from a gene and/or the expression of a peptide or protein on the surface of a cell, as the context requires. By "down-regulates" is meant that the compound acts to decrease the level of expression of the cell adhesion protein. The down-regulation may be at the level of transcription, may be by affecting translation or may act via some other mechanism, for example by effecting changes in mRNA stability.

The cell adhesion molecule to be down-regulated may be any protein that is expressed on the surface of an endothelial cell and which is capable of interaction with a leukocyte or platelet cell, such as VCAM-1, ICAM-1, LFA-1, CD2, PECAM, CD31, IAP, CD47, integrin ανβ3, MAdCAM, PECAM, selectins (P-, L- and E-selectin), LFA-3, CD80/CD86 or thrombospondin. VCAM-1 is the cell adhesion molecule of choice because interaction of VCAM-1 on ECs with its ligand, the α4β1 integrin VLA-4 on leukocytes has been shown to be the predominant mechanism triggering arrest of rolling monocytes and lymphocytes, and their subsequent spreading.

It is a feature of the present invention that the compound which prevents the expression of the cell adhesion molecule may be inducibly generated. It has been found that targeted disruption of the adhesion molecule VCAM-1 in mice is almost universally fatal for the developing embryo due to a failure of effective placentation, causing absent or delayed chorioallantoic fusion and results in the death of the embryo at between 8 and 12 days *in utero* (Gurtner *et al.,* 1995).

The ideal strategy for induction is that of conditional knockout of the cell adhesion molecule, allowing normal expression during development and in the young adult, but with the ability to inhibit expression of the cell adhesion molecule in the donor organ immediately prior to, and/or following transplantation. Suitable methods of induction will be clear to those of skill in the art, such as cloning the inhibitor compound downstream of a suitable response element, enhancer element or promoter element. For example, several known systems enable the transcription of a gene to be controlled in mammalian cells using small molecules, including the Tet-On^{TM} system (Clontech, UK), the metallothionein promoter system (Palmiter *et al.,* 1983), the ecdysone-inducible mammalian expression system (Invitrogen, BV), steroid-inducible promoters (Clackson *et al.,* 1997) and cytokine inducible promoters (Aranciba *et al.,* 1998; Bachiller *et al.,* 1990). The particular system chosen will be determined by the required degree of suppression.

The compound that down-regulates the expression of the cell adhesion molecule may be a polynucleotide. According to a second aspect of the invention there is provided a biological tissue in which the endothelial cells of the tissue may be induced to generate a polynucleotide which down-regulates the expression of a cell adhesion molecule by the cells.

The polynucleotide may be complementary in sequence to part of the gene or mRNA that encodes the cell adhesion molecule, so that it hybridises to the gene or to the mRNA and so prevents its transcription or translation. Ideally, such a polynucleotide should be of at least 15 nucleotides in length, preferably at least 50 nucleotides, more preferably greater than 100 nucleotides. To ensure that only the cell adhesion gene is targeted, the polynucleotide should be complementary to a portion of the gene that is most divergent from other nucleic acid sequences. The polynucleotide should preferably hybridise to the gene or to the mRNA encoding the cell adhesion molecule under conditions of high stringency, e.g. 0.1 x SSC, 65°C (where SSC= 0.15M NaCl, 0.015M sodium citrate, pH 7.2).

According to this aspect of the invention, the polynucleotide sequences will act to abrogate transcription of a gene encoding a cell adhesion molecule or translation of a cell adhesion mRNA by hybridising with the molecule, thereby preventing interaction of the nucleic acid with the relevant protein factors necessary for transcription or translation to take place.

Alternatively, the polynucleotide sequences may comprise a ribozyme sequence that specifically targets a gene or mRNA coding for a cell adhesion molecule.

According to a third aspect of the invention there is provided a biological tissue in which the endothelial cells of the tissue may be induced to generate a peptide or polypeptide which down-regulates the expression of a cell adhesion molecule by the cells.

The peptide or polypeptide may possess high affinity for the cell adhesion molecule. Preferably, the affinity of the compound for the cell adhesion molecule is greater that 10⁻⁸M, more preferably greater than 10⁻⁹M, even more preferably greater than 10⁻¹⁰M. The compound should also exhibit specific binding affinity for the cell adhesion molecule to ensure that binding activity of the compound it is not responsible for inappropriate destruction of other molecules in the cell.

Preferably, the compound is an antibody or antibody fragment, which may be easily prepared with high specificity and affinity for a desired target. Antibody fragments such as Fab fragments or single chain fragments (sFvs) are particularly suitable since they are small molecules with high solubility and greater penetrative capacity in an intracellular environment. Intracellular sFv antibodies have been successfully employed *in vitro* to neutralise viruses (HIV-1 (Rondon *et al.,* 1997) and flaviviruses (Jiang *et al.,* 1995)), and to decrease the expression of intracellular oncoproteins (e.g. erbB-2 (Beerli *et al.,* 1994; Graus Porta *et al.,* 1995) and ras (Bioca *et al.,* 1993)) and cell-surface receptors (e.g. IL-2R (Richardson *et al.,* 1997)). These sFv species were generated from the hybridomas of monoclonal antibodies specific for the target protein.

The polypeptide may be a fusion protein comprising a binding domain that exhibits affinity for a cell adhesion molecule and an effector domain that targets a cell adhesion molecule or that targets a gene or mRNA that codes for the cell adhesion molecule. For example, the effector domain may be delivered (by means of the binding domain) to the environment of the cell adhesion molecule, so that the adhesion molecule is targeted for destruction. If proteinaceous, the effector domain may comprise a protease, a kinase, a phosphatase or any other enzyme that is capable of inactivating a cell adhesion molecule. Alternatively, the effector domain may comprise a oligonucleotide or ribozyme molecule that acts on the gene or mRNA that codes for the cell adhesion molecule.

According to a fourth aspect of the invention there is provided a biological tissue in which the endothelial cells of the tissue may be induced to generate a bispecific fusion protein which down-regulates the expression of one or more cell adhesion molecules by the cells. Such a fusion protein may comprise domains or peptides with affinities for different cell adhesion protein epitopes. For example, a fusion protein could be designed with binding affinity and specificity against two or more different epitopes on a cell adhesion molecule such as VCAM. This would improve efficiency of knockout of the cell adhesion molecule. Furthermore, a number of epitopes could be targeted on different cell adhesion molecules, in order to simultaneously abrogate their expression.

In order that the cell adhesion molecule is not transported to the cell surface for expression, the amino acid sequence of the peptide or polypeptide must include a targeting sequence to direct degradation of the cell adhesion molecule.

The targeting sequence may comprise any suitable intracellular protein trafficking signal, provided that the signal is involved in directing the bound complex to a subcellular compartment for degradation. Examples of suitable intracellular protein trafficking signals are discussed by Pudsley, 1989 and by Magee and Wileman, 1992.

Preferably, the signal comprises a localisation signal that directs a nascent peptide to the endoplasmic reticulum (ER). A particularly suitable signal is the inclusion of the amino acid sequence Lys-Asp-Glu-Leu (KDEL) at the C-terminus of the peptide or polypeptide. Proteins that reside in the lumen of the endoplasmic reticulum (ER), the first compartment for newly made membrane-bound proteins or secreted proteins, are known to possess this short sequence (Munro and Pelham, 1987). If this sequence is deleted or extended by the addition of further amino acids, the protein is secreted from the cell rather than retained.

Alternative signal regions will be clear to those of skill in the art and include lysosomal targeting sequences. Fusion proteins may also be constructed, comprising a peptide or polypeptide fused to a viral protein such as the HIV-1 *nef* protein, or to cytoplasmic signals for rapid turnover such as are found on CTLA-4 (see Magee and Wileman, (1992) Protein targeting: a practical approach; Oxford University Press).

According to a fifth aspect of the invention there is provided a polypeptide comprising a binding region capable of binding to a cell adhesion molecule and a signalling region for subcellular targeting of the polypeptide. Preferably, the polypeptide comprises an antibody or antibody fragment, most preferably a single chain antibody fragment (sFv). The signalling region of choice is a localisation signal for the endoplasmic reticulum. Most preferably, signalling region comprises the amino acid sequence KDEL at the C terminus of the polypeptide.

According to a sixth aspect of the invention there is provided a polynucleotide encoding a peptide or polypeptide according to the fifth aspect of the invention. Preferably, the polynucleotide will comprise sequences suitable for the regulation of expression of the peptide or polypeptide. This expression can preferably be controlled, such as by cell-specific control, inducible control, or temporal control. Preferably, expression should be specific for vascular smooth muscle cells, fibroblasts, cardiac myocytes, ECs or any combination of these cell types. Most preferably, expression should be specific for ECs; EC specific expression can be achieved by using tissue-specific promoters such as E-selectin, ICAM, and MAdCAM.

According to a seventh aspect of the invention, there is provided a vector comprising a polynucleotide according to the sixth aspect of the invention.

The term "vector" signifies a moiety which is capable of transferring a polynucleotide to a host cell. Preferably the vector is a DNA vector and, more preferably, is capable of expressing RNA encoding a protein according to the invention. Numerous suitable vectors are documented in the art; examples may be found in *Molecular Cloning: a Laboratory* *Manual:* 2nd edition, Sambrook *et al.,* 1989, Cold Spring Harbor Laboratory Press or *DNA cloning: a practical approach, Volume II*: *Expression systems,* edited by D.M. Glover (IRL Press, 1995).

Many known techniques and protocols for the manipulation of nucleic acids, for example, in the preparation of nucleic acid constructs, mutagenesis, sequencing, introduction of DNA into cells and gene expression, and analysis of proteins, are described in detail in *Short Protocols in Molecular Biology,* Second Edition, Ausubel *et al.* eds., (John Wiley & Sons, 1992) or *Protein Engineering: A practical approach* (edited by A.R. Rees *et al.,* IRL Press 1993). For example, in eukaryotic cells, the vectors of choice may be virus-based.

For certain embodiments of the invention, a bicistronic expression vector can be used in order to allow stoichiometric co-expression of two genes from one mRNA. In one particular system (Jackson *et al.,* 1990), expression is driven from a single promoter and incorporation of the internal ribosome entry site (IRES) of encephalomyocarditis virus (ECMV) allows CAP-independent ribosomal binding and translation of the second open reading frame. This allows transfection of a construct containing sequences directed against two different epitopes on a cell adhesion molecule to improve efficiency of knockout, or allows the targeting of two or more different cell adhesion molecules using only one construct.

Introduction of the nucleic acid may be followed by causing or allowing expression from the nucleic acid, e.g. by culturing host cells under conditions for allowing expression of the gene.

In one embodiment, the nucleic acid of the invention is integrated into the genome (e.g. chromosome) of the host cell. Integration may be promoted by inclusion of sequences which promote recombination with the genome, in accordance with standard techniques.

Preferably the vector is suitable for the production of a transgenic animal. Vectors suitable for the generation of transgenic pigs, for example, are described in Heckl-Östreicher (1995), McCurry (1996), White (1995), Yannoutsos (1995), and Langford (1996). Minigene vectors suitable for the generation of transgenic mice are described in Diamond (1995).

According to an eighth aspect of the invention, there is provided a delivery system comprising a molecule of the fifth, sixth or seventh aspects and means to deliver said molecule to a target cell.

The delivery system may be viral or non-viral. Non-viral systems, such as liposomes, avoid some of the difficulties associated with virus-based systems, such as the expense of scaled production, poor persistence of expression, and concerns about safety. Preferably the delivery system is suitable for use in gene therapy. Numerous appropriate delivery systems are known in the art such as, for example, polycationic condensed DNA linked or unlinked to killed adenovirus alone (see Curiel, 1992) and ligand linked DNA (see Wu, 1989). Naked DNA may also be employed, optionally using biodegradable latex beads to increase uptake. Liposomes can also act as gene delivery vehicles encapsulating nucleic acid comprising a gene cloned under the control of a variety of tissue-specific or ubiquitously-active promoters. Mechanical delivery systems such as the approach described in Woffendin *el al* (1994) may also be used.

Direct delivery of gene therapy compositions will generally be accomplished, in either a single dose or multiple dose regime, by injection, either subcutaneously, intraperitoneally, intravenously or intramuscularly or delivered to the interstitial space of a tissue. Other modes of administration include oral and pulmonary administration, using suppositories, and transdermal applications, needles, and gene guns or hyposprays.

Preferably, the delivery system will be targeted so that molecules according to the present invention are taken up by cells suitable for transplantation, or cells which have been transplanted. More preferably the delivery system will be specific for these cells. For example, the delivery system may be targeted to a specific organ, such as the heart or the kidney, or to a specific cell type, such as endothelial cells.

To achieve this the delivery system may, for example, be a receptor-mediated delivery system, being targeted to receptors found on target cells. For example, the delivery system may be targeted to receptors found on heart cells, preferably to receptors found exclusively on heart cells, or it may be targeted to receptors found on endothelial cells, preferably to receptors found exclusively on endothelial cells such as E-selectin or P-selectin.

The delivery system is preferably suitable for the generation of transgenic animals. For example, the delivery system may be targeted to a gamete, a zygote, or an embryonic stem cell.

According to an ninth aspect of the invention, there is provided a method of transfecting a cell with a vector according to the invention. The cell for transfection should be a progenitor of the species which is to be the organ donor, preferably an endothelial cell. The stable transfection of porcine endothelial cells is described in Heckl-Östreicher (1995).

Preferably, the cell is suitable for the generation of a transgenic animal. More preferably, the cell is a gamete, a zygote, or an embryonic stem cell. The transfection of murine ova by microinjection to generate transgenic mice, for example, is described in Diamond (1995), and the microinjection of porcine zygotes, for instance, to generate transgenic pigs is described in Yannoutsos (1995), Langford (1996), and White (1995).

According to a tenth aspect of the invention, there is provided a cell transfected according to the ninth aspect.

According to a eleventh aspect of the invention, there is provided biological tissue comprising a cell according to tenth aspect of the invention. The term "biological tissue" as used herein includes collections of cells, tissues and organs. Accordingly the definition includes, for example, fibroblasts, nervous tissue, heart, liver, or kidney tissues or organs.

According to a twelfth aspect of the invention, there is provided an animal comprising a cell and/or biological tissue according to the invention. Preferably the animal is suitable for the production of organs for transplantation into humans. Preferably the animal is a mammal, and more preferably it is a transgenic pig or a transgenic sheep.

The animal might be treated whilst alive such that it comprises transgenic biological tissue (ie. treated by gene therapy). Preferably, a live animal is transfected with a vector which is specifically delivered to endothelial cells to produce transgenic organs suitable for xenotransplantation.

Alternatively, the animal might be bom as a transgenic animal. Various suitable approaches for generating such transgenic animals are known in the art (eg. Bradley & Liu, 1996; Clarke, 1996; Wheeler, 1994). For example, direct manipulation of the zygote or early embryo, by microinjection of DNA for instance, is well known, as is the *in vitro* manipulation of pluripotent cells such as embryonic stem cells. Retroviral infection of early embryos has proved successful in a range of species, and adenoviral infection of zona-free eggs has been reported. Transgenesis and cloning of sheep by nuclear transfer has also been described (eg. WO97/07668).

According to a thirteenth aspect of the invention, there is provided a method of rendering biological tissue suitable for transplantation, comprising expressing one or more compounds according to the present invention in the biological tissue, preferably exclusively in its endothelial cells. The biological tissue may be so rendered either *in vivo* or *ex vivo.* For example, an animal organ may be *in vivo* transfected with a vector according to the invention, or an organ could be transfected *ex vivo* before transplantation or *in vivo* after transplantation.

According to an fourteenth aspect of the invention, there is provided a method of transplantation comprising transplanting biological tissue according to the invention from a donor animal into a recipient animal. Preferably the method is for xenotransplantation and the donor biological tissue is xenogeneic with respect to the recipient animal.

The invention will now be described in detail with specific reference to single chain antibody fragments directed against the VCAM-1 molecule. It will be appreciated that modification of detail may be made without departing from the scope of the invention.

### BRIEF DESCRIPTION OF THE FIGURES

Figure 1: Leukocyte-Endothelial Cell Adhesion cascade
Figure 2: Flow cytometry with phage-antibodies demonstrating specificity for VCAM
Figure 3: Restriction enzyme fingerprinting of the sFv clones
Figure 4: Expression vector for sFv
Figure 5: Cotransfection of sFv/ER with pEF/GFP/ER
Figure 6a: VCAM expression on stable EC transfectants with sFv clone F5
Figure 6b: VCAM expression on stable EC transfectants with sFv clone E6.2
Figure 7a: VCAM expression on stable EC transfectants with sFv clone F5
Figure 7b: VCAM expression on stable EC transfectants with sFv clone E6.2
Figure 8: Staining for VCAM in untransfected and transfected endothelial cells
Figure 9: Binding of Jurkat cells to varying densities of endothelial cells

### EXAMPLES

### Example 1: Identification of VCAM-specific sFv from a phage-display library

A phage-display antibody library was used to generate an sFv specific for VCAM-1. The library used contains >10⁸ clones generated using a bank of 50 cloned human VH gene segments with a random nucleotide sequence encoding CDR3 lengths of 4-12 residues (Richardson *et al.,* 1993). This library has already been used to isolate specific single-chain antibodies to a variety of antigens including haptens, foreign and self antigens. However selection has depended upon the availability of purified or recombinant antigen. We developed a novel screening strategy to overcome the lack of recombinant porcine VCAM.

cDNA for porcine VCAM was used to generate stable cell lines that exhibit high levels of surface porcine VCAM expression as assessed by flow cytometry. VCAM positive cells were incubated with 3µM CellTracker^{TM} Green CMFDA (5-chloromethylfluorescein diacetate, (Molecular Probes, Oregon) for 1 hour at 37°C. Once this membrane-permeant probe enters the cell esterase hydrolysis converts non-fluorescent CMFDA to fluorescent 5-chloromethylfluorescein which reacts with thiols on proteins to form aldehyde-fixable conjugates. Cells labelled with this are viable and fluorescent for several cell divisions.

A suspension of VCAM-negative cells were incubated with the phage-library in 4% Marvel/PBS at 4°C with gentle agitation. After 30 minutes, the labeled VCAM-positive cells were then added in a 1:10 ratio (VCAM positive:negative) and incubated for a further 90 minutes. The cells were pelleted by centrifugation and washed with PBS three times. The fluorescent VCAM-positive cells (and phage that was bound to the cell surface) were then separated by FACS. Phage were 'rescued' in the standard manner (Griffiths *et al.,* 1994), amplified and the library was subjected to three further rounds of screening. "Polyclonal" phage ELISA confirmed that the library was enriched for VCAM-specific phage. Individual clones were then tested by flow cytometry for binding to the VCAM-positive cell line and the results from representative clones are shown in Figure 2.

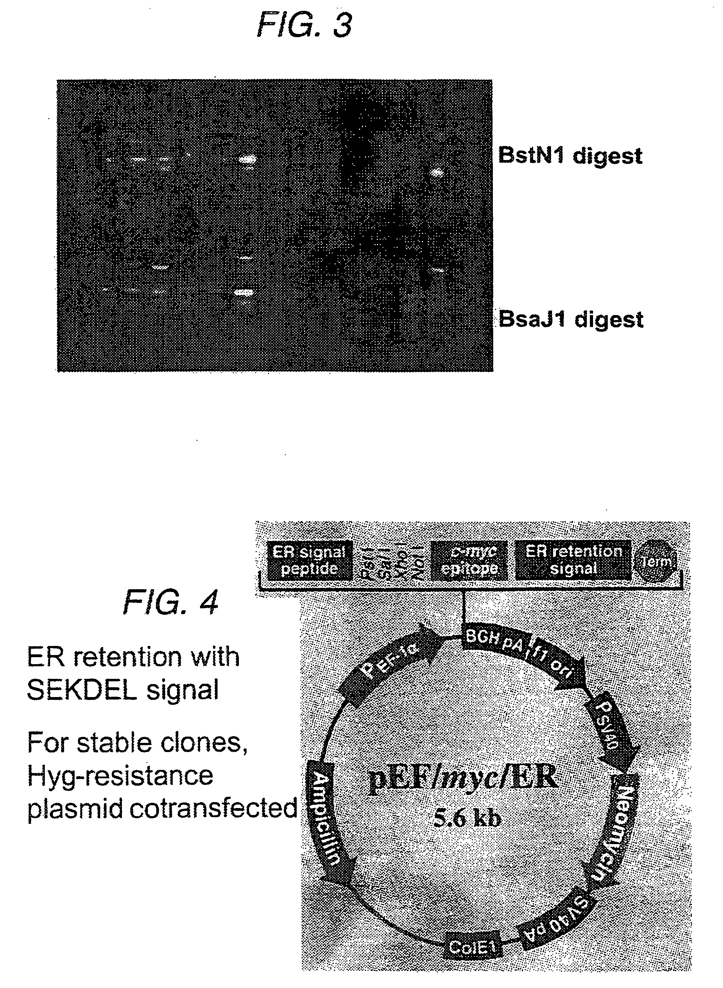

The sequence of the VCAM-specific sFv clones was amplified from minipreps of the phagemid vector by PCR and digested for 18 hours with either *Bst*N1 or *Bsa*J1 according to the manufacturer's protocol. Restriction fragment length polymorphism (RFLP) mapping of the first 15 VCAM-specific clones showed that there were at least 5 distinct patterns of digestion suggesting at least 5 different antibody sequences. (Figure 3). Two of these were used for further analysis.

### Example 2: Subcloning of sFv for targeted intracellular expression

Our strategy was to engineer the VCAM-specific sFv to be retained within the ER, so that providing that the sFv-VCAM interaction was of sufficient affinity, both molecules would be retained within the ER and degraded, thereby reducing cell-surface VCAM levels. Initially, the sFv has been expressed using a constitutively active promoter, the promoter from the human elongation factor 1α-subunit (EF-1α).

The sFv was amplified from the phagemid vector by PCR (30 cycles, annealing temperature 55°C, 1.5µM Mg²⁺) using the primers: (5')CAGTCTATGCGGCCCCATTCA(3'); and

(5')TCCACAGGCGCGCACTCCCAGCCGGGCATGGCCCAGGT(3').

The resulting fragment was subcloned into *Bss*HII/*Not*l sites in pEF/*myc*/ER (Invitrogen, BV). The sFv was directed to the ER by incorporation of the sequence of a signal peptide from a mouse VH chain at the 5'-end of the sFv gene; this peptide is cleaved upon translocation into the ER. The sFv is retained because of the KDEL peptide sequence at the C-terminus. The construct is shown diagramatically in Figure 4.

### Example 3: Effect of sFv constructs on VCAM expression

### Co-transfection of sFv/ER with pEF/GFP/ER

Functional analysis of the constructs was carried out by transfection into an immortalized porcine endothelial cell line A9. These were generated by microinjection of pZipSVU19 DNA into primary aortic endothelial cells (Dorling *et al.,* 1996). The immortalized cells retain the characteristics of endothelial cells but unlike primary ECs, demonstrated constitutive expression of VCAM. Cytokine treatment of the cells increased VCAM expression marginally (RMFI increase from 38.2 to 66.3 at 72 hours).

Transfection of DNA was carried out with the liposome formulation LipofectAMINE (Life Technologies) using a modification of the manufacturer's protocol. LipofectAMINE reagent is a 3:1 (w/w) formulation of the polycationic lipid 2,3-dioleyloxy-N-[2(sperminecarboxamido)ethyl]-N,N-dimethyl-1-propanaminium trifluoroacetate (DOSPA) and the neutral lipid dioleoyl phosphatidtlethanolamine (DOPE) in water.

1 x 10⁵ cells were seeded in each well of a 6-well plate in complete medium (DMEM) and allowed to attach overnight at 37°C, in a 5% CO₂ incubator overnight. The following day the cells were washed twice in pre-warmed serum-free Opti-MEM® I (Gibco BRL). 2 mL of serum-free Opti-MEM® I was added to each well and the cells returned to the 37°C CO₂ incubator for 2-3 hours.

DNA to be transfected (1-2µg per transfection) was diluted to a final volume of 100µL per transfection in serum-free medium, Opti-MEM® I. To a separate tube, for each transfection, 5µL LiopfectAMINE and 15µg Bovine Transferrin (Sigma; stock solution made up as 1 µg/µL in serum-free Opti-MEM® I) were added to a final volume of 100µL in serum-free Opti-MEM® I. The DNA and the LipofectAMINE-transferrin mix were combined and left to stand at room temperature for 30 minutes to allow DNA-liposome complex formation.

For each transfection, 0.8mL of prewarmed serum-free Opti-MEM® I was added to the tube containing the complexes. The medium was aspirated from the walls and 1mL of the diluted complex solution was added to the cells. The cells were incubated at 37°C in 5% CO₂ for 5-6 hours. The transfection mixture was then removed and 2mL of complete medium (DMEM) containing 10% FCS added to each well.

In transient transfection assays, the cells were assayed 24-72 hours after the start of transfection. The transfected cells were harvested and stained with the monoclonal antibody 10.2C7 specific for porcine VCAM (Celltech, UK) and a second layer reagent labeled with Texas red. In order to identify cells which had taken up the plasmid DNA, the sFv constructs were co-transfected with the vector pEF/GFP/ER which contains a 716bp fragment from pαGFP (Crameri *et al.,* 1996) fused to the KDEL ER-retention signal in the same vector backbone as the sFv constructs.

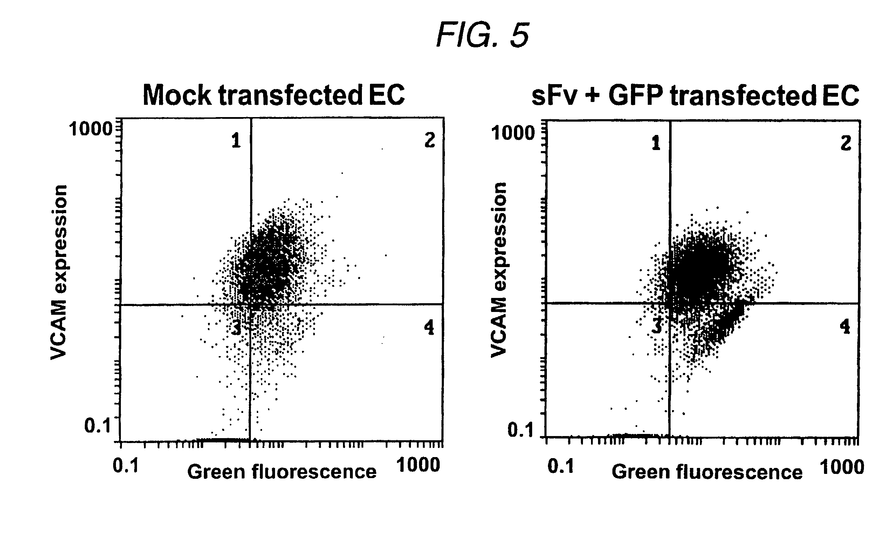

Figure 5 shows a typical histogram when the population was assessed for VCAM expression (red fluorescence) and GFP expression (green fluorescence). Cells expressing GFP showed a 77% reduction in VCAM expression (RMFI control 10.9, GFP positive cells 2.4). This reduction was not seen in cells transfected with the pEF/GFP/ER plasmid alone suggesting the reduction was due to the expression of the VCAM-specific sFv.

### Example 4: Analysis of stable transfectants

To obtain stable transfectants, IPEC were co-transfected with a plasmid encoding hygromycin resistance (Kioussis *et al.,* 1987). After 48-72 hours the cells were passaged and diluted 1:10 into complete medium containing 150µg/mL hygromycin. VCAM expression was analyzed by flow cytometry of the cells resistant to hygromycin at 10-14 days.

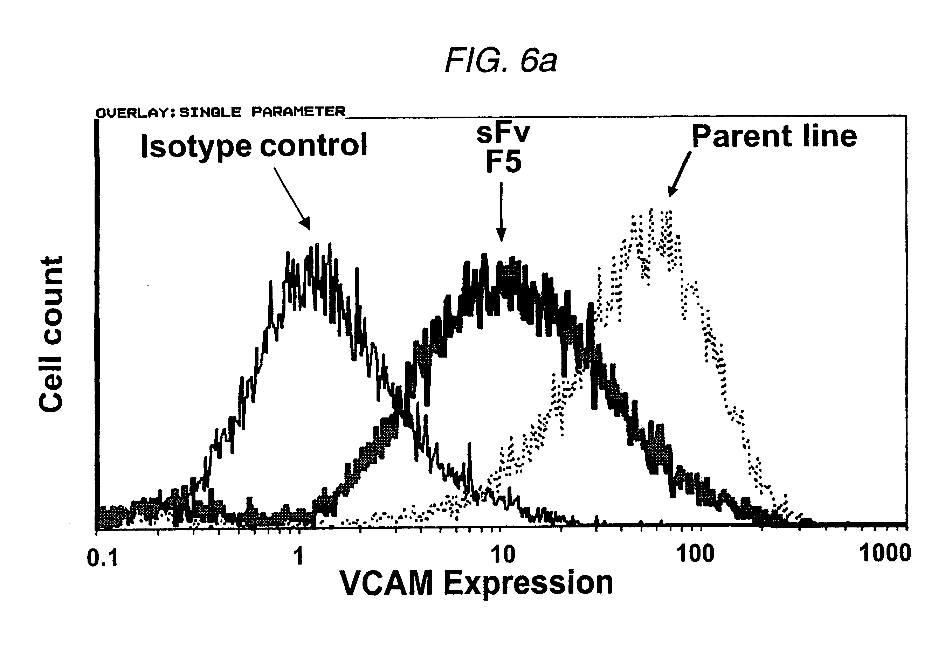

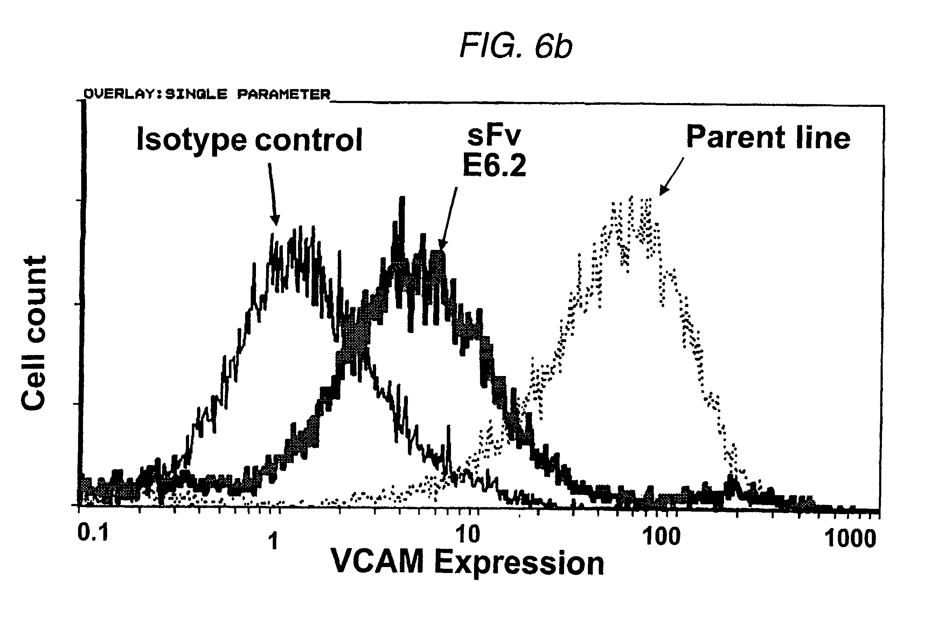

Figures 6 A and B show typical histograms of cell lines generated by transfection of constructs of two different sFv clones, E6.2 and F5. Both sFv constructs reduce VCAM expression on the surface of the endothelial cells. The values for mean fluorescence corresponding to the level of VCAM expression demonstrated in these Figures are: RMFI untransfected population = 38.2, clone sFv/ER E6.2 = 4.82 and F5 = 10.1.

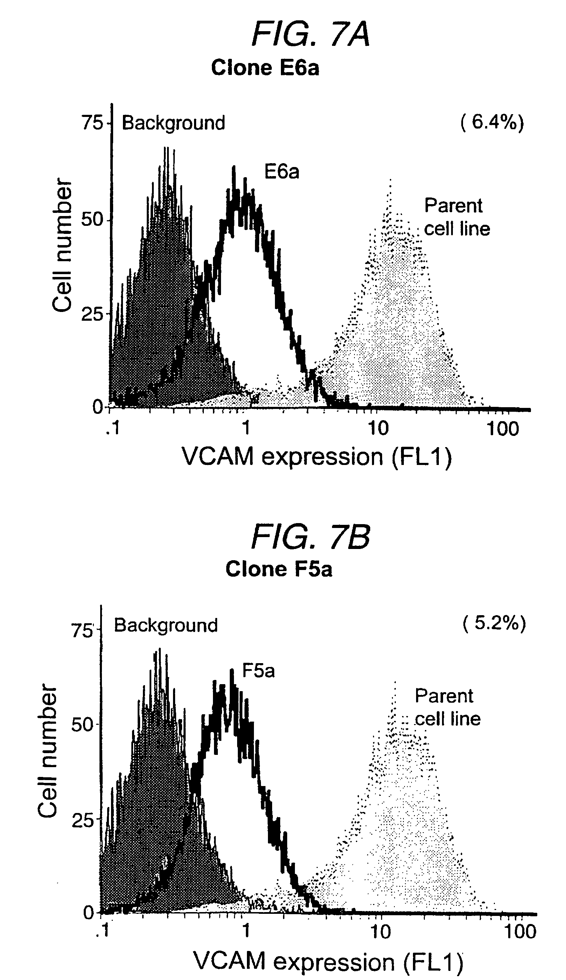

Figures 7 A and B present the results of a second experiment in permanently transfected cells to assess the reduction of VCAM expression on the surface of these cells.

These data demonstrate that it is possible to reduce VCAM expression on endothelial cells by expressing within the cells a VCAM-specific scFv targeted for retention within the ER.

### Example 5: Transfection of scFv-ER constructs traps VCAM within the cell.

The techniques of immunofluorescence staining and confocal microscopy were then used to demonstrate co-localisation of VCAM and scFv within both the E6.2 and F5 scFv transfectants.

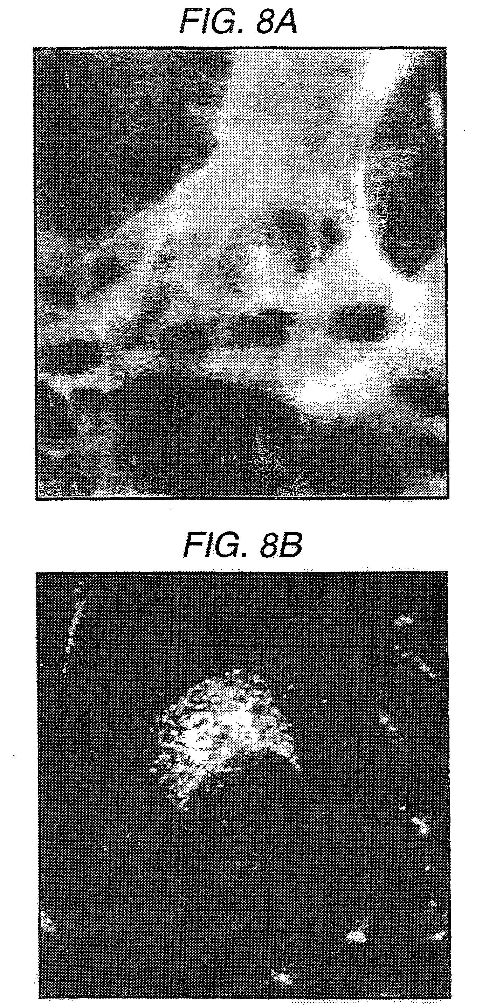

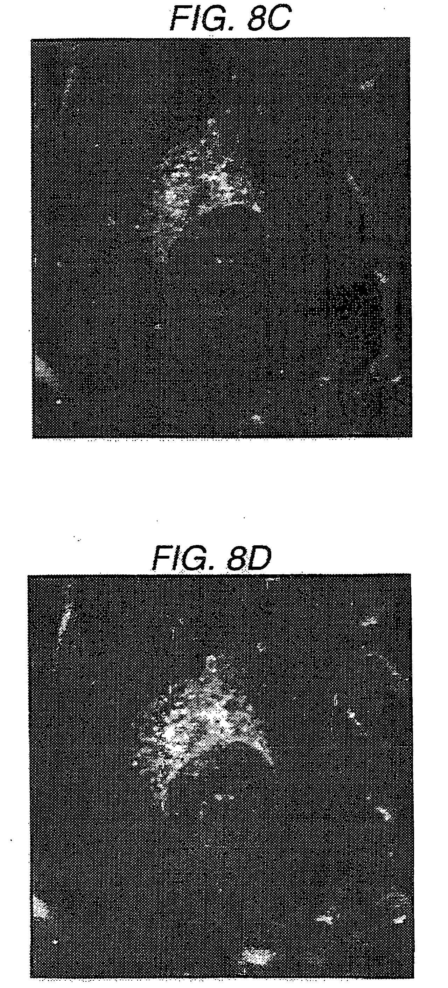

Untransfected A9 endothelial cells demonstrate diffuse membrane staining for VCAM (see Figure 8A). Both scFv transfected clones demonstrated a perinuclear, punctuate staining pattern when stained for VCAM expression, consistent with retention of VCAM within the endoplasmic reticulum (an example of one transfected cell is shown in Figure 8B). Staining the transfected cells with an anti-myc antibody to detect the c-myc epitope on the scFv constructs produced a strong perinuclear staining pattern consistent with ER-retention of the scFv (Figure 8C). Dual exposure with appropriate filters confirmed that the VCAM and scFv were co-localised in the ER (Panel D).

### Example 6: Reduction of VCAM expression by intracellular scFv is associated with reduction in adherence of human leukocytes

In order to demonstrate that reduction in VCAM expression achieved by the intracellular scFv was functionally significant, binding of the T cell leukaemia line, Jurkat, was examined, both to the transfectants and to the parent A9 endothelial cell line.

The Jurkat line J6 was chosen because of high expression of surface VLA-4 and documented reliance on this integrin for binding to endothelial cells (van Kooyk, Y *et al;* 1993).

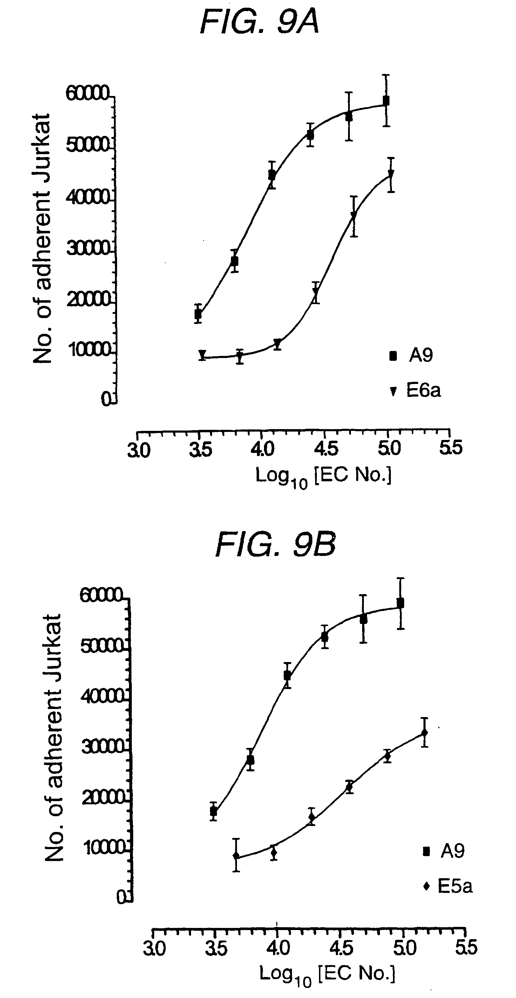

The graphs shown in Figures 9A and B demonstrate the binding of Jurkat cells to varying densities of endothelial cells plated in individual wells of a 96 well plate. For both scFv transfectants, there was a right shift in the binding curve demonstrating a reduced affinity of the Jurkat for the transfectants, as well as a significant reduction in the maximal binding of the leukocytes to the endothelial cell monolayer.

### REFERENCES

Alon, R., P. D. Kassner, M. W. Carr, E. B. Finger, M. E. Hemler, and T. A. Springer. 1995. The integrin VLA-4 supports tethering and rolling in flow on VCAM-1. *J-Cell-Biol 128:1243-53 issn: 0021-9525.*
Aranciba-Carcamo *et al.,* (1998) **65(1):** 62-7.
Bach, F. H. 1998. Xenotransplantation: problems and prospects. *Annu Rev Med 49:301-10*.
Bachiler *et al.,* (1990) Arch Geschwulstforsch, **60(5):** 357-60.
Beerli, R. R., W. Wels, and N. E. Hynes. 1994. Intracellular expression of single chain antibodies reverts ErbB-2 transformation. *J-Biol-Chem 269: 23931-6.*
Bevilacqua, M. P. 1993. Endothelial-leukocyte adhesion molecules. *Annu Rev Immunol 11:767-804.*
Biocca, S., P. Pierandrei Amaldi, and A. Cattaneo. 1993. Intracellular expression of anti-p21ras single chain Fv fragments inhibits meiotic maturation of xenopus oocytes. *Biochem-Biophys-Res-Commun 197:422-7.*
Blakely, M. L., W. J. Van der Werf, M. C. Berndt, A. P. Dalmasso, F. H. Bach, and W. W. Hancock. 1994. Activation of intragraft endothelial and mononuclear cells during discordant xenograft rejection. *Transplantation 58:1059-66 issn: 0041-1337.*
Bradley A, Liu P. Target practice in transgenics. *Nature Genet.* 1996; 14: 121-123.
Brown, E. J., and F. P. Lindberg. 1996. Leucocyte adhesion molecules in host defence against infection. *Ann-Med 28:201-8 issn: 0785-3890.*
Clackson, T. 1997. Controlling mammalian gene expression with small molecules. *Curr. Opin. Chem. Biol. 1:210-8.*
Clarke AR. The adenovirus and the egg: a new approach to transgenesis. *Nature Biotech.* 1996; 14: 942.
Crameri, A., E. A. Whitehom, E. Tate, and W. P. Stemmer. 1996. Improved green fluorescent protein by molecular evolution using DNA shuffling. *Nat-Biotechnol 14:315-9.*
Curiel (1992) *Hum Gene Ther* 3:147-154
Diamond LE, McCurry KR, Oldham ER, Tone M, Waldmann H, Platt JL, Logan JS. Human CD59 expressed in transgenic mouse hearts inhibits the activation of complement. *Transplant Immunol.* 1995; 3: 305-312.
Dorling, A., and R. I. Lechler. 1994. Prospects for xenografting. *Curr-Opin-Immunol 6:765-9 issn: 0952-7915.*
Dorling, A., C. Stocker, T. Tsao, D. O. Haskard, and R. I. Lechler. 1996. In vitro accommodation of immortalized porcine endothelial cells: resistance to complement mediated lysis and down-regulation of VCAM expression induced by low concentrations of polyclonal human IgG antipig antibodies. *Transplantation 62:1127-36 issn: 0041-1337.*
Foster, C. A. 1996. VCAM-1/alpha 4-integrin adhesion pathway: therapeutic target for allergic inflammatory disorders. *J-Allergy-Clin-Immunol 98:S270-7 issn: 0091-6749.*
Graus Porta, D., R. R. Beerli, and N. E. Hynes. 1995. Single-chain antibody-mediated intracellular retention of ErbB-2 impairs Neu differentiation factor and epidermal growth factor signaling. *Mol-Cell-Biol 15*:*1182-91.*
Griffiths, A. D., S. C. Williams, O. Hartley, I. M. Tomlinson, P. Waterhouse, W. L. Crosby, R. E. Kontermann, P. T. Jones, N. M. Low, T. J. Allison, *and et al.* 1994. Isolation of high affinity human antibodies directly from large synthetic repertoires. *EMBO-J 13:3245-60.*
Gurtner, G. C., V. Davis, H. Li, M. J. McCoy, A. Sharpe, and M. I. Cybulsky. 1995. Targeted disruption of the murine VCAM1 gene: essential role of VCAM-1 in chorioallantoic fusion and placentation. *Genes-Dev 9:1-14 issn: 0890-9369.*
Heckl-Ostreicher B, Binder R, Kirschfink M. Functional activity of the membrane-associated complement inhibitor CD59 in a pig-to-human *in vitro* model for hyperacture xenograft rejection. *Clin. Exp. Immunol.* 1995;102:589-595.
Isobe, M., H. Yagita, K. Okumura, and A. Ihara. 1992. Specific acceptance of cardiac allograft after treatment with antibodies to ICAM-1 and LFA-1. *Science 255:1125-7 issn: 0036-8075.*
Jiang, W., K. Venugopal, and E. A. Gould. 1995. Intracellular interference of tick-borne flavivirus infection by using a single-chain antibody fragment delivered by recombinant Sindbis virus. *J-Virol 69:1044-9.*
Jones, D. A., L. V. McIntire, C. W. Smith, and L. J. Picker. 1994. A two-step adhesion cascade for T cell/endothelial cell interactions under flow conditions. *J-Clin-Invest 94:2443-50 issn: 0021-9738.*
Kioussis, D., F. Wilson, C. Daniels, C. Leveton, J. Taverne, and J. H. Playfair. 1987. Expression and rescuing of a cloned human tumour necrosis factor gene using an EBV-based shuttle cosmid vector. *EMBO-J 6:355-61.*
Langford GA, Cozzi E, Yannoutsos N, Lancaster R, Elsome K, Chen P, White DGJ. Production of pigs transgenic for human regulators of complement activation using YAC technology. *Transplant Proc.* 1996; 28: 862-863.
Magee, A., and T. Wileman. 1992. Protein Targeting: A Practical Approach. In *Practical Approach Series.* Oxford University Press, Oxford.
McCurry KR, Diamond LE, Kooyman DL, Byme GW, Martin MJ, Logan JS, Platt JL. Human complement regulatory proteins expressed in transgenic swine protect swine xenografts from humoral injury. *Transplant Proc.* 1996; 28: 758.
Millan, M. T., C. Geczy, K. M. Stuhlmeier, D. J. Goodman, C. Ferran, and F. H. Bach. 1997. Human monocytes activate porcine endothelial cells, resulting in increased E-selectin, interleukin-8, monocyte chemotactic protein-1, and plasminogen activator inhibitor-type-1 expression. *Transplantation 63:421-9 issn: 0041-1337.*
Mueller, J. P., M. J. Evans, R. Cofiell, R. P. Rother, L. A. Matis, and E. A. Elliott. 1995. Porcine vascular cell adhesion molecule (VCAM) mediates endothelial cell adhesion to human T cells. Development of blocking antibodies specific for porcine VCAM. *Transplantation 60:1299-306 issn: 0041-1337.*
Muller, W. A. 1995. The role of PECAM-1 (CD31) in leukocyte emigration: studies in vitro and in vivo. *J-Leukoc-Biol 57:523-8 issn: 0741-5400.*
Munro, S., and H. R. Pelham. 1987. A C-terminal signal prevents secretion of luminal ER proteins. *Cell 48:899-907 *LHM: This title is held by RPMS library *LHC: Vol. 19 (1980)-.*
Palmiter *et al.,* 1983 *Science,* **222**: (4625): 809-814.
Pudsley, A. 1989. *Protein Targeting.* Academic Press, San Diego, CA.
Richardson, J. H., T. A. Waldmann, J. G. Sodroski, and W. A. Marasco. 1997. Inducible knockout of the interleukin-2 receptor alpha chain: expression of the high-affinity IL-2 receptor is not required for the in vitro growth of HTLV-I-transformed cell lines. *Virology 237:209-16.*
Rondon, I. J., and W. A. Marasco. 1997. Intracellular antibodies (intrabodies) for gene therapy of infectious diseases. *Annu Rev Microbiol 51:257-83.*
Springer, T. A. 1994. Traffic signals for lymphocyte recirculation and leukocyte emigration: the multistep paradigm. *Cell 76:301-14 issn: 0092-8674.*
Tanaka, Y., D. H. Adams, and S. Shaw. 1993. Proteoglycans on endothelial cells present adhesion-inducing cytokines to leukocytes. *Immunol-Today 14:111-5 issn: 0167-4919.*
Tedder, T. F., D. A. Steeber, A. Chen, and P. Engel. 1995. The selectins: vascular adhesion molecules. *FASEB-J 9:866-73 issn: 0892-6638.*
van Kooyk,Y et al; 1993 "Lymphocyte function-associated antigen 1 dominates very late antigen 4 in binding of activated T cells to endothelium. *J Exp Med 177: 185-90.*
Wheeler MB. Development and validation of swine embryonic stem cells: a review. *Reprod Fertil. Dec.* 1994; 6:563-568.
White D, Cozzi E, Langford G, Oglesby T, Wang M, Wright L, Wallwork J. The control of hyperacute rejection by genetic engineering of the donor species. *Eye* 1995; 9:185-189.
Woffendin *et al* (1994) *Proc. Natl. Acad. Sci. USA* 91(24):11581-11585
Wu (1989) *J Biol Chem* 264:16985-16987
Yannoutsos N, Langford GA, Cozzi E, Lancaster R, Elsome K, Chen P, White DJG. Production of pigs transgenic for human regulators of complement activation. *Transplant Proc.* 1995; 27: 324-325.

## Claims

1. A biological tissue comprising endothelial cells which may be induced to generate a compound which down-regulates the expression of a cell adhesion molecule by the cells, the compound being either (a) a polynucleotide complementary in sequence to pan of the gene or mRNA that encodes the cell adhesion molecule, (b) a polynucleotide comprising a ribozyme sequence that specifically targets a gene or mRNA that encodes the cell adhesion molecule, or (c) a peptide or polypeptide with specific binding affinity for the cell adhesion molecule.

2. A tissue according to claim 1, wherein said polypeptide (c) is a bispecific fusion protein.

3. A polypeptide comprising a binding region capable of binding to a cell adhesion molecule and a signalling region for subcellular targeting of the polypeptide such that it is not transported to the cell surface.

4. A polypeptide according to claim 3, which comprises an antibody or antibody fragment.

5. A polypeptide according to claim 4, which comprises a single chain Fv fragment.

6. A polypeptide according to any of claims 3 to 5, wherein the signalling region for subcellular targeting of the polypeptide comprises a localisation signal for the endoplasmic reticulum.

7. A polypeptide according to claim 6, wherein the signaling region comprises the amino acid sequence KDEL at the C terminus of the polypeptide.

8. A polypeptide according to any one of claims 3 to 7, wherein said binding region has affinity for any one of the adhesion molecules VCAM-1, ICAM-1, LFA-1, CD2, PECAM, CD31, 1AP, CD47 or integrin αvβ3.

9. A polynucleotide encoding a polypeptide according to any one of claims 3 to 8.

10. A vector comprising a polynucleoride according to claim 9.

11. A cell comprising a polynucleotide according to claim 9 or a vector according to claim 10.

12. Biological tissue comprising a cell according to claim 11.

13. A non-human animal comprising biological tissue according to claim 12 and/or a cell according to claim 11.

14. An animal according to claim 13, wherein said animal is a transgenic pig or sheep.

15. A method of rendering a tissue or organ suitable for transplantation, comprising expressing a polypeptide according to any one of claims 3 to 8 in endothelial cells in said tissue or organ, thereby down-regulating the expression of a cell adhesion molecule.

16. Biological tissue according to claim 12, for use in transplantation.

17. The use of biological tissue according to claim 12 in the manufacture of a medicament for transplanting into a recipient animal.

18. The use of claim 17, wherein said recipient animal is a human.

## Patentansprüche

1. Ein endotheliale Zellen enthaltendes biologisches Gewebe, welches induziert werden kann, eine Zusammensetzung zu erzeugen, die die Exprimierung und/oder Freisetzung eines Zelladhäsionsmoleküles durch die Zellen herunterreguliert, wobei die Zusammensetzung entweder (a) ein Polynucleotid ist, das mit der Sequenz zu dem Teil von dem Gen oder der mRNA, welches das Zelladhäsionsmolekül kodiert, komplementär ist, oder (b) ein Polynucleotid ist, dass eine Ribozym-Sequenz enthält, die spezifisch auf ein Gen oder eine mRNA zielt, welche ein Zelladhäsionsmolekül kodieren, oder (c) ein Peptid oder Polypeptid mit spezifischer Bindungsaffinität für das Zelladhäsionsmolekül ist.

2. Ein Gewebe nach Anspruch 1, wobei das genannte Polypeptid (c) ein bispezifisches Fusionsprotein ist.

3. Ein Polypeptid, das eine Bindungsregion enthält, die in der Lage ist, an ein Zelladhäsionsmolekül zu binden, und das eine Signalregion für eine subzelluläre Zielsteuerung des Polypeptids enthält, so dass es nicht an die Zelloberfläche transportiert wird.

4. Ein Polypeptid nach Anspruch 3, dass einen Antikörper oder ein Antikörperfragment enthält.

5. Ein Polypeptid nach Anspruch 4, das ein einzelkettiges Fv-Fragment enthält.

6. Ein Polypeptid nach einem der Ansprüche 3 bis 5, wobei die Signalregion für die subzelluläre Zielsteuerung des Polypeptids ein Lokalisationssignal für das endoplasmatische Reticulum enthält.

7. Ein Polypeptid nach Anspruch 6, wobei die Signalregion am C-Terminus des Polypeptids die Aminosäuresequenz KDEL enthält.

8. Ein Polypeptid nach einem der Ansprüche 3 bis 7, wobei die genannte Bindungsregion eine Affinität für eines der Adhäsionsmoleküle VCAM-1, ICAM-1, LFA-1, CD2, PECAM, CD31, IAP, CD47 oder Integrin αvβ3 aufweist.

9. Ein Polynucleotid, das ein Polypeptid nach einem der Ansprüche 3 bis 7 kodiert.

10. Ein Vektor, der ein Polynucleotid nach Anspruch 9 enthält.

11. Eine Zelle, die ein Polynucleotid nach Anspruch 9 oder einen Vektor nach Anspruch 10 enthält.

12. Ein biologisches Gewebe, das eine Zelle nach Anspruch 11 enthält.

13. Ein nichtmenschliches Tier, das ein biologisches Gewebe nach Anspruch 12 und/oder eine Zelle nach Anspruch 11 enthält.

14. Ein Tier nach Anspruch 13, wobei das genannte Tier ein transgenes Schwein oder Schaf ist.

15. Ein Verfahren um ein Gewebe oder Organ für eine Transplantation geeignet zu machen, **dadurch gekennzeichnet, dass** ein Polypeptid nach einem der Ansprüche 3 bis 8 in endothelialen Zellen im genanntem Gewebe oder Organ exprimiert und/oder freigesetzt wird, und hierdurch die Exprimierung und/oder Freisetzung eines Zelladhäsionsmoleküles herunterreguliert wird.

16. Verwendung eines biologischen Gewebes nach Anspruch 12 für Transplantationen.

17. Verwendung eines biologischen Gewebes nach Anspruch 12 bei der Herstellung eines Arzneimittels für das Verpflanzen in ein Empfänger-Tier.

18. Verwendung von Anspruch 17, wobei das genannte Empfänger-Tier ein Mensch ist.

## Revendications

1. Un tissu biologique constitué de cellules endothéliales pouvant être induites de manière à générer un composé qui régule négativement l'expression d'une molécule d'adhésion cellulaire par les cellules, le composé étant soit (a) un polynucléotide dont la séquence est complémentaire à une partie du gène ou de l'ARNm qui code la molécule d'adhésion cellulaire, (b) un polynucléotide constitué d'une séquence ribosomale qui cible spécifiquement un gène ou un ARNm qui code pour la molécule d'adhésion cellulaire, ou (c) un peptide ou polypeptide qui a une affinité de liaison spécifique pour la molécule d'adhésion cellulaire.

2. Un tissu suivant la revendication 1, dans laquelle le polypeptide (c) est une protéine de fusion bi-spécifique.

3. Un polypeptide constitué d'une région de fixation capable de se fixer à une molécule d'adhésion cellulaire et une région signal pour le ciblage subcellulaire d'un polypeptide de manière telle qu'il n'est pas transporté à la surface.

4. Un polypeptide suivant la revendication 3, qui comprend un anticorps ou un fragment d'anticorps.

5. Un polypeptide suivant la revendication 4, qui comprend un fragment Fv simple chaîne.

6. Un polypeptide suivant l'une quelconque des revendication 3 à 5, dans laquelle la région signal pour le ciblage subcellulaire du polypeptide comprend un signal pour la localisation dans le réticulum endoplasmique.

7. Un polypeptide suivant la revendication 6, dans laquelle la région signal comprend la séquence aminoacide KDEL à l'extrémité C du polypeptide.

8. Un polypeptide suivant l'une quelconque des revendications 3 à 7, dans laquelle la région de fixation a une affinité pour l'une quelconque des molécules d'adhésion VCAM-1, ICAM-1, LFA-1, CD2, PECAM, CD31, 1AP, CD47 ou l'intégrine αvβ3.

9. Un polynucléotide codant pour un polypeptide suivant l'une quelconque des revendications 3 à 8.

10. Un vecteur comprenant un polynucléotide suivant la revendication 9.

11. Une cellule comprenant un polynucléotide suivant la revendication 9 ou un vecteur suivant la revendication 10.

12. Un tissu biologique comprenant une cellule suivant la revendication 11.

13. Un animal non humain ayant du tissu biologique suivant la revendication 12 et/ou une cellule suivant la revendication 11.

14. Un animal suivant la revendication 13, dans laquelle cet animal est un cochon ou un mouton transgénique.

15. Un procédé qui rend un tissu ou un organe acceptable pour la greffe, consistant en l'expression d'un polypeptide suivant l'une quelconque des revendications 3 à 8 dans des cellules endothéliales du tissu ou de l'organe, avec comme conséquence la régulation négative de l'expression d'une molécule d'adhésion cellulaire.

16. Un tissu biologique suivant la revendication 12, destiné à être utilisé dans une greffe.

17. L'utilisation d'un tissu biologique suivant la revendication 12 dans la fabrication d'un médicament destiné à la greffe dans un animal hôte.

18. L'utilisation de la revendication 17, dans laquelle l'animal hôte est un humain.
